# EUROPÄISCHE PATENTSCHRIFT

(11) **EP 2 155 661 B1**
(45) Veröffentlichungstag und Bekanntmachung des Hinweises auf die Patenterteilung: **10.09.2014**
(21) Anmeldenummer: 08760004.5
(22) Anmeldetag: 26.05.2008
(51) Int. Cl.: C07C 231/06, C07C 253/00

(54) **VERFAHREN ZUR HERSTELLUNG VON NITRILEN**
PROCESS FOR PREPARING NITRILES
PROCÉDÉ DE FABRICATION DE NITRILES

(30) Priorität: 31.05.2007 EP 07109303
(43) Veröffentlichungstag der Anmeldung: 24.02.2010
(73) Patentinhaber: BASF SE, 67056 Ludwigshafen (DE)
(72) Erfinder: ERNST, Martin, 69115 Heidelberg (DE); GITTER, Markus, 01099 Dresden (DE)
(86) Internationale Anmeldenummer: PCT/EP2008/056405
(87) Internationale Veröffentlichungsnummer: WO 2008/145626

(56) Entgegenhaltungen:
- WO-A-98/31657
- WO-A-2006/083924
- WO-A-2006/085685
- GB-A- 570 835
- US-A- 3 940 429
- US-A- 3 983 161

## Beschreibung

Die vorliegende Erfindung betrifft ein Verfahren zur Herstellung von Nitrilen bzw. Iminen oder Enaminen durch Umsetzung von primären und/oder sekundären Aminen in der Gasphase in Gegenwart von Sauerstoff.

Nitrile sind wichtige Ausgangsmaterialien und Zwischenprodukte in der Synthese von funktionellen Verbindungen, wie Pharmazeutika, Pflanzenschutzmitteln, Lichtschutzmitteln, Stabilisatoren, oder Polymeren. So kann die Cyano-Gruppe der Nitrile beispielsweise zu Aminen, Amiden, Carbonsäuren, Aldehyden, Alkoholen oder Ketonen umgesetzt werden. Daneben kann das in α-Position zur Cyano-Gruppe stehende Kohlenstoffatom alkyliert werden. Typische Reaktionen, die die Cyano-Gruppe oder das in α-Position zur Cyano-Gruppe stehende Kohlenstoffatom betreffen, sind beispielsweise im Ullmann beschrieben (Ullmann Encyclopedia of Industrial Chemistry, "Nitriles", Kapitel 2.2, Wiley-VCH-Verlag, Weinheim, 2005).

Nitrile werden in der Regel durch Umsetzung von Olefinen, Alkoholen oder Aldehyden mit Ammoniak dargestellt. Daneben können Nitrile auch aus stickstoffhaltigen Vorläufern, wie Aminen, Amiden oder Formamiden, durch Dehydrogenierung bzw. Oxidation hergestellt werden. Weitere Syntheserouten beinhalten die Addition von Blausäure an Doppelbindungen.

Insbesondere die Dehydrogenierung bzw. Oxidation von Aminen ermöglicht die Synthese von interessanten Zwischenprodukten für Pharmazeutika, Pflanzenschutzmitteln, Lichtschutzmitteln oder Stabilisatoren.

Feldhues et al. (U. Feldhues, H. J. Schäfer, Synthesis (1982), 145) beschreiben einen elektrochemischen Prozess zur Dehydrogenierung von Nitrilen in wässriger Lösung.

Mizuno et al. (K. Yamaguchi, N. Mizuno, Angew. Chem. Int. Ed., 42 (2003), 1480-1483) offenbaren ein Verfahren zur Oxidation von Aminen zu Nitrilen oder Iminen mit Sauerstoff in Gegenwart eines auf Aluminiumoxid geträgerten Rutheniumkatalysators. Das Verfahren wird in dem Lösungsmittel Trifluortoluol durchgeführt.

S. Uemura et al. (Y. Maeda, T. Nishimura, S. Uemura, Bull. Chem. Soc. Jpn., 76 (2003), 2399-2403) veröffentlichten eine Synthesemethode für Nitrile ausgehend von Aminen an Kupferkatalysatoren unter Verwendung von Molsieben. Als Lösungsmittel wurde Toluol verwendet.

Eine Synthese von Nitrilen durch Oxidation von Aminen an Ruthenium-Hydroxylapatit-Katalysatoren mit Luftsauerstoff wurde von Kaneda et al. beschrieben (K. Mori, K. Yamaguchi, T. Mizugaki, K. Erbitani, K. Kaneda, Chem. Comm., (2001), 461-462). Die Reaktion wurde in aprotischen Lösungsmitteln, wie Toluol, durchgeführt.

US 3,983,161 und US 3,940,429 offenbaren die Umsetzung von ungesättigten Aminen mit Sauerstoff zu den entsprechenden Nitrilen in Gegenwart von Kupferhalogeniden in einer Lösung, die eine Stickstoffbase enthält.

GB 570,835 offenbart die Herstellung organischer Nitrile durch Oxidation ungesättigter Amine, welche mindestens eine, durch eine Einfachbindung am Amin-Stickstoff gebundene Methylengruppe aufweisen, mit Sauerstoff an einem Oxidationskatalysator bei einer Temperatur von mindestens 450°C.

In der WO 2006/085685 wird die Herstellung von 3-Amino-2,2-dimethylpropionitril aus dem korrespondierenden Azidonitril beschrieben.

Die Durchführung von Oxidationsreaktionen in Gegenwart von organischen Lösungsmitteln beinhaltet ein potentielles Explosionsrisiko, so dass bei einer großtechnischen Ausführung solcher Verfahren ein hoher Sicherheitsaufwand betrieben werden muss. Weiterhin müssen die Reaktionsprodukte in der Regel von dem verwendeten Lösungsmittel in aufwändigen Aufarbeitungsschritten abgetrennt werden.

Der vorliegenden Erfindung lag deshalb die Aufgabe zu Grunde, ein verbessertes Verfahren zur Herstellung von Nitrilen, Iminen und/oder Enaminen aus Aminen bereitzustellen. Insbesondere sollten ein Verfahren zur Verfügung gestellt werden, das ausreichend kurze mittlere Verweilzeiten aufweist und somit auch kontinuierlich durchgeführt werden kann. Weiterhin sollte das Verfahren technisch sicher und ökonomisch umgesetzt werden können. Eine hohe Verfahrensökonomie sollte zudem durch einfache Aufarbeitungsschritte bzw. einer Rückführung von Edukten sichergestellt werden.

Es wurde gefunden, dass die Umsetzung von Alkylamin, Cycloalkylamin, Dicycloalkylamin, Alkoxyamin, Alkandiamin, Cycloalkyldiamin oder Alkoxydiamin mit Sauerstoff in der Gasphase durchgeführt werden kann.

Gegenstand der Erfindung ist demnach Verfahren zur Herstellung von Nitrilen, Iminen und/oder Enaminen durch Umsetzung eines primären und/oder sekundären Amins, welches ein Alkylamin, Cycloalkylamin, Dicycloalkylamin, Alkoxyamin, Alkandiamin, Cycloalkyl-diamin oder Alkoxydiamin ist, in Gegenwart von Sauerstoff und eines Oxidationskatalysators, dadurch gekennzeichnet, dass man ein Amin in die Gasphase überführt und in einem sauerstoffhaltigen Gasstrom bei einer Temperatur von 100 bis 350°C über einen Oxidationskatalysator leitet.

Das erfindungsgemäße Verfahren lässt sich diskontinuierlich oder bevorzugt kontinuierlich durchführen.

Üblicherweise wird das Verfahren in Festbettreaktoren durchgeführt, in denen der Katalysator als Festbett im Reaktor angeordnet ist. Beispiele für geeignete Festbettreaktoren finden sich in Ullmann's Encyclopedia of Industrial Chemistry, "Fixed-Bed Reactors", Wiley-VCH-Verlag, Weinheim, 2005.

Vorzugsweise wird das Verfahren in Rohrreaktoren, Rohrbündelreaktoren oder einer Monostranganlage durchgeführt. Bei einer Monostranganlage besteht der Rohrreaktor aus einer Hintereinanderschaltung mehrerer (z.B. zweier oder dreier) einzelner Rohreaktoren.

Das Verfahren kann auch in Wirbelbettreaktoren durchgeführt werden, in denen das Katalysatormaterial sich einer auf- und abwirbelnder Bewegung befindet.

Das erfindungsgemäße Verfahren wird in der Regel so ausgeführt, dass die Edukte (Amine) in die Gasphase überführt werden und in einem sauerstoffhaltigen Gasstrom über einen Oxidationskatalysator geleitet werden.

Zur Überführung in die Gasphase, wird das Amin üblicherweise verdampft.

Die Verdampfung erfolgt in der Regel mittels eines Verdampfers. Dabei kann flüssiges Amin direkt verdampft werden, oder das Amin kann in Form einer wässrigen Lösung zusammen mit Wasser verdampft werden.

Flüssige Amine können auch in die Gasphase überführt werden, indem ein Gasstrom durch das flüssige Amin geleitet wird. Dabei wird der Gasstrom in der Regel partiell oder vollständig mit Amin gesättigt. Weiterhin ist es auch möglich, dass der Gasstrom durch eine wässrige Lösung eines Amins geleitet wird, wobei der Gasstrom in der Regel neben dem Amin teilweise Wasserdampf enthält. Vorzugsweise wird der Gasstrom und/oder das Amin bzw. die wässrige Aminlösung erwärmt, um die Aufnahmekapazität des Gasstroms an Amin zu erhöhen. Der Gasstrom kann beispielsweise mittels eines Gaseinleitungsrohrs, eines Verteilerringes oder einer Düse in das flüssige Amin bzw. die wässrige Aminlösung eingeleitet werden.

Wird das Amin in Form einer wässrigen Lösung eingesetzt, so liegt die AminKonzentration der wässrigen Lösung üblicherweise im Bereich zwischen 1 bis 90 Gew.-%, vorzugsweise 5 bis 70 Gew.-% und besonders bevorzugt 10 bis 50 Gew.-%.

Der Gasstrom enthält Sauerstoff. Neben Sauerstoff, enthält der Gasstrom üblicherweise Inertgase, wie Stickstoff oder Edelgase, wie Helium, Neon, Argon, Xenon oder Krypton. Üblicherweise wird Sauerstoff in Form von Luft eingesetzt. Der Volumenanteil an Luft im Gasstrom kann in einem breiten Bereich variieren. Üblicherweise beträgt der Volumenanteil von Luft im Gasstrom im Bereich von 1 bis 100 Vol.-%, vorzugsweise 5 bis 80 Vol.-% und besonders bevorzugt 30 bis 70 Vol.-%. Vorzugsweise setzt sich der Gasstrom zu gleichen Volumenteilen aus Luft und einem Inertgas, insbesondere Stickstoff, zusammen.

Der Sauerstoff kann aber auch in elementarer Form, d.h. als Gas mit einem Sauerstoffgehalt von mehr 95 Vol.-%, vorzugsweise mehr als 98 Vol.-% und besonders bevorzugt mehr als 99 Vol.-%, in das Verfahren eingesetzt werden.
Der Sauerstoffanteil im Gasstrom liegt in der Regel im Bereich von 0,2 bis 50 Vol.-%, vorzugsweise 1 bis 40 Vol.-% und besonders bevorzugt 5 bis 20 Vol.-%.

Das Verfahren wird üblicherweise in einem Druckbereich von 0,1 bis 4 MPa (1 bis 40 bar), bevorzugt 0,1 bis 2,5 MPa, besonders bevorzugt 0,1 bis 1 MPa, und einem Temperaturbereich von 100 bis 350°C, bevorzugt 120 bis 250°C, besonders bevorzugt 150 bis 220°C, durchgeführt.

Die mittlere Verweilzeit im Reaktor liegt in der Regel im Bereich von 0,01 bis 100 Sekunden, bevorzugt im Bereich von 0,1 bis 50 Sekunden und besonders bevorzugt im Bereich von 0,5 bis 10 Sekunden.
Bei der Durchführung des Verfahrens in einem Festbettreaktor kann das Katalysatorfestbett sowohl von oben als auch von unten angeströmt werden.
Bevorzugt wird das Verfahren in einer Kreisgasfahrweise durchgeführt.
Die Katalysatorbelastung in einem Festbettreaktor liegt im allgemeinen im Bereich von 0,01 bis 10 kg Amin pro Liter Katalysator (Schüttvolumen) und Stunde, bevorzugt im Bereich von 0,05 bis 5 kg Amin pro Liter Katalysator und Stunde und besonders bevorzugt im Bereich von 0,1 bis 2 kg Amin pro Liter Katalysator und Stunde.

Das eingesetzte Amin wird vorzugsweise durch den Sauerstoff im Gasstrom zu den entsprechenden Nitrilen, Iminen und/oder Enaminen oxidiert.

Die Isolierung der gebildeten Reaktionsprodukte kann beispielsweise durch Einleitung des Gasstroms in ein inertes Lösungsmittel (Quench) erfolgen.

Als Lösungsmittel für den Quench eignen sich eine Vielzahl organischer Lösungsmittel. Beispiele umfassen aliphatische, cyclische oder acyclische Kohlenwasserstoffe, insbesondere cyclische und acyclische, verzweigte oder unverzweigte Alkane oder Alkangemische, beispielsweise n-Pentan, i-Pentan, Cyclopentan, Hexan, Cyclohexan, Heptan, Octan und Petrolether, aromatische Kohlenwasserstoffe, beispielsweise Benzol, Toluol und Xylole, chlorierte aromatische Kohlenwasserstoffe, beispielsweise Chlorbenzol, Alkohole, beispielsweise Methanol, Ethanol, Ethylenglykol, 1,4-Butandiol und Polyetheralkohole, insbesondere Polyalkylenglykole, beispielsweise Diethylenglykol und Dipropylenglykol, aliphatische Nitrile, beispielsweise Acetonitril und Propionitril, Ether, beispielsweise Dioxan, THF, Diethylether und Ethylenglykoldimethylether sowie Gemische der vorstehend aufgeführten Lösungsmittel. Als Lösungsmittel für den Quench wird vorzugsweise ein aliphatischer Kohlenwasserstoff oder ein Polyalkylenglykol, insbesondere ein gesättigter cyclischer oder acyclischer, aliphatischer Kohlenwasserstoff mit 5 bis 8 C-Atomen, beispielsweise Pentan, Hexan, Cyclohexan, Heptan, Monoethylenglykol, Dipropylenglykol, oder 1,4-Butandiol verwendet.

Es ist auch möglich, die Reaktionsprodukte durch Auskondensierung aus dem Gasstrom zu isolieren. Die Auskondensierung kann beispielsweise dadurch erfolgen, dass der Gasstrom an einem Kühler oder Kondensator auf Temperaturen unterhalb des Siedepunktes des Amins abgekühlt werden.

Zweckmäßigerweise kann der Reaktionsaustrag, wenn unter erhöhtem Druck gearbeitet wurde, vor der Isolierung der Reaktionsprodukte entspannt werden.

Die Reaktionsprodukte können mittels den bekannten Aufreinigungsmethoden, wie Destillation bzw. Rektifikation, Flüssigextraktion, Phasenseparation oder Auskristallisation von den Ausgangsmaterialien und ggf. Wasser oder den Flüssigkeiten, die zum Quench- bzw. in der Extraktion eingesetzt wurden, abgetrennt und gereinigt werden.

Nichtreagierte Ausgangsprodukte, sowie der sauerstoffhaltige Gasstrom können in die Reaktionszone zurückgeführt werden.

Als im erfindungsgemäßen Verfahren einsetzbare primäre und/oder sekundäre Amine eignen sich praktisch alle Alkylamine, Cycloalkylamine, Dicycloalkylamine, Alkoxyamin, Alkandiamine, Cycloalkyldiamine und Alkoxydiamine sowie deren Mischungen. Sie können geradkettig, verzweigt oder cyclisch sein.

Vorzugsweise werden die Amine in flüssiger Form in das Verfahren eingesetzt. Feste Amine können beispielsweise durch Erwärmen in die flüssige Form überführt werden. Wasserlösliche oder partiell wasserlösliche Amine können auch als wässrige Lösung eingesetzt werden, wobei die Aminkonzentration üblicherweise im Bereich zwischen 1 bis 90 Gew.-%, vorzugsweise 5 bis 70 Gew.-% und besonders bevorzugt 10 bis 50 Gew.-%. liegt.

Die Amine können ferner Substituenten tragen oder funktionelle Gruppen enthalten, welche sich unter den Bedingungen der Oxidation inert verhalten, beispielsweise Alkoxy-, Alkylamino- oder Dialkylaminogruppen, Ester- oder Säuregruppen, Halogene oder Amidgruppen.

### Beispiele für Amine sind:

Alkylamine, wie C₁- bis C₁₀-Alkylamine oder C₂- bis C₂₀-Dialkylamine, beispielsweise Methylamin, Dimethylamin, Ethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentyl-amin, n-Hexylamin, s-Hexylamin, iso-Hexylamin, n- Octylamin, 2-Ethylhexylamin, Di-n-Hexylamin oder Di(2-Ethylhexyl)amin,
Cycloalkylamine, wie C₅- bis C₈-Cycloalkylamine, beispielsweise Cyclopentylamin oder Cyclohexylamin,
Dicycloalkylamine, wie C₁₀- bis C₁₆-Dicycloalkylamine, beispielsweise Dicyclohexylamin,
Alkoxyamine, wie 3-Methoxypropylamin, 2-Ethoxyethylamin, 3-Ethoxypropylamin, 3-(2-Ethylhexyloxy)propylamin, 1-(2-Hydroxyethyl)-piperazin oder Polyetheramine, Alkandiamine, wie C₂- bis C₂₀-Alkandiamine, beispielsweise Ethylendiamin, 1,3-Propandiamin, 1,2-Propandiamin, Neopentyldiamin, Hexamethylendiamin, Octamethylendiamin, 3-(Methylamino)propylamin, 2-(Diethylamino)ethylamin, 3-(Dimethylamino)propylamin, 3-(Diethylamino)propylamin oder 2,2-Dimethylpropandiamin, Cycloalkyldiamine, wie C₅- bis C₁₆-Cycloalkyldiamine, beispielsweise Isophorondiamin, Cyclohexyldiamin, 3-(Cyclohexylamino)propylamin, Diaminodicyclohexylmethan oder 3,3'-Dimethyl-4,4'-diaminodicyclohexylmethan,
Alkoxydiamine, wie 4,9-Dioxadecan-1,12-diamin, 4,7,10-Trioxatridecan-1,13-diamin oder 3-(Methylamino)propylamin,
Es ist auch möglich chirale Amine einzusetzen, wie

(R)-3-Methyl-2-butylamin, (S)-3-Methyl-2-butylamin, (R)-2-Hexylamin, (S)-2-Hexylamin, (R)-2-Heptylamin, (S)-2-Heptylamin, (R)-2-Octylamin, (S)-2-Octylamin, (R)-2-Nonylamin, (S)-2-Nonylamin, (R)-3,3-Dimethyl-2-aminobutan, (S)-3,3-Dimethyl-2-aminobutan, (R)-1-Cyclohexylethylamin, (S)-1-Cyclohexylethylamin oder (S)-1-Methoxy-2-aminopropan.

In einer bevorzugten Ausführungsform werden Alkandiamine, Cycloalkyldiamine oder Alkoxydiamine, wie oben beschrieben, beispielsweise C₄- bis C₁₀-Alkandiamine, wie Neopentyldiamin, Hexamethylendiamin, Octamethylendiamin, 3-(Methylamino)propylamin, 2-(Diethylamino)ethylamin, 3-(Dimethylamino)propylamin, 3-(Diethylamino)propylamin oder 2,2-Dimethylpropylendiamin, in das Verfahren eingesetzt. Besonders bevorzugt wird 2,2-Dimethylpropylendiamin in das Verfahren eingesetzt.
In der bevorzugten Ausführungsform werden Alkandiamine, Cycloalkyldiamine oder Alkoxydiaminevorzugsweise zu Aminonitrilen umgesetzt.

Die so erhaltenen Aminonitrile können zu den entsprechenden Aminoamiden hydrolysiert werden. So kann beispielsweise 2,2-Dimethylpropylendiamin zu 3-Amino-2,2-dimethyl-propionitril oxidiert werden, welches zu 3-Amino-2,2-dimethyl-propionsäureamid hydrolysiert werden kann.

Aminoamide lassen sich beispielsweise durch Umsetzung eines Aminonitrils mit einer starken Mineralsäure in Gegenwart von Wasser und einem organischen Lösungsmittel, in welchem das erhaltende Salz des Amids ausfällt, gemäß der in WO-A1-98331657 beschriebenen Weise herstellen. Hierzu wird das Aminonitril in der Regel in einem Lösungsmittel gelöst. Geeignete Lösungsmittel sind beispielsweise Dialkylether, wie Diethylether; Dialkylethylenglykole, wie Ethylenglykoldimethylether, Ethylenglykoldiethylether, Ethylenglykoldibutylether, Ethylenglykolmethylbutylether und Ethylenglykolpropylethylether; sekundäre Alkohole, wie Isopropanol; und/oder Kohlenwasserstoffe, wie Heptan und Hexan. Als Lösungsmittel sollte üblicherweise ein Lösungsmittel gewählt werden, in dem das Aminonitril ausreichend löslich ist, so dass es durch die zugegebene Säure hydrolysiert werden kann und in dem das durch die Reaktion gebildete Salz des Aminoamids unlöslich oder nur teilweise löslich ist, so dass das gebildete Aminoamid-Salz von der Reaktionslösung abgetrennt werden kann. Die Ausbeute an Aminoamid ist in der Regel je höher, desto geringer die Löslichkeit des gebildeten Ethylenglykoldimethylether. Zu der Lösung aus Aminonitril und Lösungsmittel werden üblicherweise 0,5 bis 4 Moläquivalente Wasser, bezogen auf die Nitrilgruppe, zugegeben. Vorzugsweise beträgt das Molverhältnis von Wasser zu Nitrilgruppen 1:1. Ein hoher Überschuss an Wasser kann zur Bildung von Aminocarbonsäuren führen. Die Reaktionsmischung wird in der Regel auf Temperaturen im Bereich von 0 bis 50°C, vorzugsweise 0 bis 30°C, besonders bevorzugt 0 bis 15°C abgekühlt. Üblicherweise erfolgt dann unter Kühlung die Zugabe einer starken Mineralsäure, wobei die Temperatur vorzugsweise 30°C nicht übersteigen sollte, um Nebenreaktionen zu vermeiden. Als Mineralsäuren kommen üblicherweise HCl, HBr, H₂SO₄, Toluolsulfonsäure oder Trifluoressigsäure in Betracht. Bevorzugt wird die Mineralsäure in wasserfreier Form zugegeben. Das Molverhältnis von Mineralsäure zu Aminonitril liegt üblicherweise in einem Bereich von 1:1 bis 6:1, wobei ein Bereich von 3:1 bis 4:1 bevorzugt ist. Nach Beendigung der Mineralsäurezugabe wird die Reaktionsmischung üblicherweise erwärmt, da die Reaktion ansonsten nur langsam verläuft. Bevorzugt wird die Reaktionsmischung für einen Zeitraum von 4 bis 20 Stunden auf Temperaturen von 40°C und mehr erwärmt. Weiterhin ist es bevorzugt die Erwärmung bei einem erhöhten Druck, beispielsweise in einem geschlossenen System vorzunehmen, um den Verlust von Säure über die Gasphase zu minimieren. Das Aminoamid fällt in der Regel in Form seines Salzes aus und kann nach Beendigung der Reaktion vom Reaktionsgemisch abgetrennt und isoliert werden. Üblicherweise wird das Salz durch Filtration abgetrennt und mit Wasser gewaschen. Das erhaltene Aminoamid-Salz wird in der Regel durch Zugabe von basischen Substanzen zum Aminoamid umgesetzt. Als basische Substanzen kommen beispielsweise Ammoniumhydroxid oder Alkalihydroxide, wie Natriumhydroxid oder Kaliumhydroxid in Betracht.

Aminoamide können beispielsweise auch durch Umsetzung von Aminonitrilen mit Wasserstoffperoxid in Gegenwart von katalytischen Mengen Natriummolybdat erhalten werden (Y. Ohtsuka, J. Org. Chem. Vol. 44, No. 5, 1979). Die Umsetzung erfolgt in der Regel durch Zugabe einer wässrigen Natriummolybdatlösung und einer wässrigen Wasserstoffperoxidlösung zu einer Lösung oder Suspension des Aminonitrils in einem Lösungsmittel. Als Lösungsmittel können beispielsweise die vorstehend genannten Lösungsmittel verwendet werden, vorzugsweise wird Ethanol verwendet. Die Konzentration des Aminonitrils in dem eingesetzten Lösungsmittel beträgt in der Regel 0,01 bis 0,1 mol/l, vorzugsweise 0,04 bis 0,06 mol/l. Die Konzentration der wässrigen Natriummolybdatlösung beträgt üblicherweise 0,1 bis 1 Gew.-%, vorzugsweise 0,4 bis 0,6 Gew.-%. Als Wasserstoffperoxidlösung wird üblicherweise eine 30-prozentige Lösung von Wasserstoffperoxid in Wasser (Perhydrol) eingesetzt. Das Volumenverhältnis von Natriummolybdatlösung zu Perhydrol beträgt in der Regel 1,5:1 bis 2,5:1, vorzugsweise 2:1. Das Volumenverhältnis von Natriummolybdatlösung zu dem eingesetzten Lösungsmittel beträgt üblicherweise 1:5 bis 1:20, vorzugsweise 1:10 bis 1:15. Die Lösung oder Suspension des Aminonitrils wird zusammen mit der zugegebenen Natriummolybdatlösung und Wasserstoffperoxidlösung üblicherweise in einem Temperaturbereich von 20 bis 50°C, vorzugsweise Raumtemperatur, gerührt, wobei die Reaktionsdauer üblicherweise im Bereich von 10 bis 20 Stunden liegt. Der ausgefallene Niederschlag wird in der Regel nach Beendigung der Reaktion durch Filtration isoliert und üblicherweise mit Wasser gewaschen.

Die so erhaltenen Aminonitrile bzw. Aminoamide können als Zwischenprodukten für Pharmazeutika, Pflanzenschutzmitteln, Lichtschutzmitteln oder Stabilisatoren eingesetzt werden.

In das erfindungsgemäße Verfahren werden Oxidationskatalysatoren eingesetzt.

Als Oxidationskatalysatoren kommen im allgemeinen heterogene Oxidationskatalysatoren in Betracht, wie Metalloxide, komplexe Mehrkomponentenoxide, Metalle oder Metalllegierungen. Beispiele für heterogene Oxidationskatalysatoren und deren Herstellung finden sich beispielsweise in Ullmann's Encyclopedia of Industrial Chemistry, "Heterogeneous Catalysis and Solid Catalysts", Kapitel 3 und 4, Wiley-VCH-Verlag, Weinheim, 2005.
Vorzugsweise werden Metalloxide als Oxidationskatalysatoren in das erfindungsgemäße Verfahren eingesetzt.

Beispiele für metallische Oxidationskatalysatoren sind die Elemente Pt, Pd, Rh, Ir und Ru. Metallische Oxidationskatalysatoren können beispielsweise in Form von Metallnetzen oder Gittern eingesetzt werden. In der Regel werden metallische Oxidationskatalysatoren eingesetzt, die durch Tränkung (Imprägnierung) von Trägermaterialien erhalten wurden (getränkte Katalysatoren).

Die Trägermaterialien können beispielsweise in Form von Pulvern oder Formkörpern, wie Strängen, Tabletten, Kugeln oder Ringen, eingesetzt werden. Für Wirbelbettreaktoren geeignetes Trägermaterial wird vorzugsweise durch Sprühtrocknung erhalten. Als Trägermaterialien kommen beispielsweise Aluminiumoxid (Al₂O₃), Titandioxid (TiO₂), Zirkoniumdioxid (ZrO₂), Siliziumdioxid (SiO₂), Magnesiumoxid (MgO), Zinkoxid (ZnO) oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der oben genannten Elemente in Betracht. Im Anschluss wird das getränkte Trägermaterial in der Regel getrocknet und ggf. calciniert.

Zum Erhalt von metallischen Oxidationskatalysatoren wird das getränkte Tägermaterial nach der Trocknung bzw. Calcinierung üblicherweise bei erhöhten Temperaturen, in der Regel in einem Bereich von 50 bis 300°C, mit Wasserstoff reduziert. Der Wasserstoffdruck liegt im Allgemeinen in einem Bereich von 1 bis 300 bar.

Die Tränkung kann auch nach der sogenannten "incipient wetness"-Methode erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermatierial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

Als Oxidationskatalysatoren kommen auch Metalloxide in Betracht. Bevorzugte Oxidationskatalysatoren sind Metalloxide der Elemente Cu, Zn, V, Nb, Mo, Re, Fe und Cr, wie CuO, ZnO, V₂O₇, Nb₂O₅, MoO₃, Re₂O₇, Fe₂O₃ und Cr₂O₃.

Metalloxide können ungeträgert oder auf Trägermaterialien aufgebracht in das Verfahren eingesetzt werden. Oxidationskatalysatoren auf Basis von Metalloxiden können durch Tränkung erhalten werden. In der Regel werden Oxidationskatalysatoren auf Basis von Metalloxiden durch Fällungsreaktionen, wie Mischfällung oder Auffällung, hergestellt (Fällungskatalysatoren).

So können Oxidationskatalysatoren über eine gemeinsame Fällung (Mischfällung) aller ihrer Komponenten hergestellt werden. Dazu wird in der Regel ein lösliches Metallsalz der entsprechenden Metalloxide und ggf. eine lösliche Verbindung eines Trägermaterials in einer Flüssigkeit in der Wärme und unter Rühren so lange mit einem Fällungsmittel versetzt, bis die Fällung vollständig ist.
Als Flüssigkeit wird in der Regel Wasser eingesetzt.

Als lösliche Metallsalze der entsprechenden Metalloxide kommen üblicherweise die entsprechenden Nitrate, Sulfate, Acetate oder Chloride der Elemente Cu, Zn, V, Nb, Mo, Re, Fe und Cr in Betracht.

Als wasserlösliche Verbindungen eines Trägermaterials werden in der Regel wasserlösliche Verbindungen von Al, Ti, Zr, Si, Mg oder Zn, beispielsweise die wasserlöslichen Nitrate, Sulfate, Acetate oder Chloride dieser Elemente, verwendet.

Oxidationskatalysatoren können weiterhin durch Auffällung hergestellt werden.
Unter Auffällung wird eine Katalysatorherstellung verstanden, bei der ein schwer lösliches oder unlösliches Trägermaterial in einer Flüssigkeit suspendiert wird, und nachfolgend lösliche Metallsalze der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Aluminiumoxid (Al₂O₃), Titandioxid (TiO₂), Zirkoniumdioxid (ZrO₂), Siliziumdioxid (SiO₂), Magnesiumoxid (MgO) oder Zinkoxid (ZnO) oder deren Gemische in Betracht.
Das Trägermaterial liegt in der Regel als Pulver oder Splitt vor.
Als Flüssigkeit, in der das Trägermaterial suspendiert wird, wird üblicherweise Wasser eingesetzt.

Als lösliche Metallsalze der entsprechenden Metalloxide kommen in der Regel die entsprechenden Nitrate, Sulfate, Acetate oder Chloride der Elemente Cu, Zn, V, Nb, Mo, Re, Fe und Cr in Betracht.

Bei den Fällungsreaktionen ist im Allgemeinen die Art der verwendeten löslichen Metallsalze nicht kritisch. Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zur Störungen führen, sei es, indem sie unerwünschte Fällungsreaktionen verursachen oder indem sie durch Komplexbildung die Fällung erschweren oder verhindern.

Üblicherweise werden bei den Fällungsreaktionen die löslichen Verbindungen durch Zugabe eines Fällungsmittels als schwer- oder unlösliche, basische Salzen gefällt. Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100°C, besonders 30 bis 90°C, insbesondere bei 50 bis 70°C, durchgeführt werden.

Die bei den Fällungsreaktionen erhaltenen Niederschläge sind im allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im allgemeinen bei 80 bis 200°C, vorzugsweise 100 bis 150°C, getrocknet und anschließend calciniert.

Die Calcinierung wird im allgemeinen bei Temperaturen zwischen 300 und 800°C, vorzugsweise 400 bis 600°C, insbesondere bei 450 bis 550°C ausgeführt.

Nach der Calcinierung werden die Fällungskatalysator üblicherweise konditioniert. Die Konditionierung kann beispielsweise dadurch erfolgen, dass man den Fällungskatalysator durch Vermahlen auf eine bestimmte Korngröße einstellt.
Nach der Vermahlung kann der Fällungskatalysator mit Formhilfsmitteln wie Graphit, oder Stearinsäure vermischt werden und zu Katalysatorformkörpern weiterverarbeitet werden.

Gängige Verfahren der Formgebung sind beispielsweise im Ullmann [Ullmann's Encyclopedia Electronic Release 2000, Kapitel: 'Catalysis and Catalysts', S28-32] und von Ertl et al. [Ertl, Knözinger, Weitkamp: "Handbook of Heterogenoeous Catalysis", VCH Weinheim, 1997, Seiten 98 ff] beschrieben.

Wie in den genannten Literaturstellen beschrieben, können durch den Prozess der Formgebung Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Für Wirbelschichtreaktoren geeignete Katalysatoren werden vorzugsweise nach der Vermahlung aus einer Suspension des vermahlenen Katalysators durch Sprühtrocknung erhalten.

Nach der Konditionierung bzw. Formgebung erfolgt in der Regel eine Temperung. Die Temperaturen bei der Temperung entsprechen üblicherweise den Temperaturen bei der Calcinierung.

Die auf diese Weise hergestellten Fällungskatalysatoren enthalten die katalytisch aktiven Komponenten in Form eines Gemisches ihrer sauerstoffhaltigen Verbindungen, d.h. insbesondere als Oxide, Mischoxide und/oder Hydroxide. Die hergestellten Fällungskatalysatoren können als solche gelagert werden.

Weiterhin kommen als Oxidationskatalysatoren komplexe Mehrkomponentenoxide, beispielsweise Zeolithe, Vanadiumphosphate und Aluminiumsilikate, in Betracht. Weitere Beispiele für komplexe Oxidationskatalysatoren finden sich beispielsweise im Ullmann (Ullmann's Encyclopedia of Industrial Chemistry, "Heterogeneous Catalysis and Solid Catalysts", Kapitel 3.1.1.2, Wiley-VCH-Verlag, Weinheim, 2005).

Die Aktivität von Oxidationskatalysatoren kann mit zunehmender Standzeit abnehmen. Die Wiederherstellung der Aktivität (Regenerierung) kann beispielsweise durch Behandlung der Katalysatoren mit einer Flüssigkeit erfolgen. Die Behandlung des Katalysators mit einer Flüssigkeit soll dazu führen, dass eventuell anhaftende Verbindungen, die aktive Stellen des Katalysators blockieren, abgelöst werden. Die Behandlung des Katalysators mit einer Flüssigkeit kann durch Rühren des Katalysators in einer Flüssigkeit oder durch Waschen des Katalysators in der Flüssigkeit erfolgen, wobei nach erfolgter Behandlung die Flüssigkeit durch Filtration oder Abdekantieren zusammen mit den abgelösten Verunreinigungen vom Katalysator abgetrennt werden kann. Geeignete Flüssigkeiten sind in der Regel das Produkt der Hydrierung, Wasser oder ein organisches Lösungsmittel, bevorzugt Ether, Alkohole oder Amide.

Die Regenerierung der Katalysatoren kann vorzugsweise auch durch Tempern der Katalysatoren unter erhöhter Temperatur, in der Regel 300 bis 900°C, erfolgen.

Das Tempern wird bevorzugt unter einer oxidierenden Atmosphäre, in der Regel in Gegenwart von Sauerstoff bzw. Luft, durchgeführt, so dass am Katalysator anhaftende organische Verbindungen zu flüchtigen Verbindungen wie CO₂ oxidiert werden.

Ein Vorteil des erfindungsgemäßen Verfahrens zur Herstellung von Aminen, Iminen und/oder Enaminen gegenüber dem Stand der Technik ist, dass in der Regel keine organischen Lösungsmittel eingesetzt werden. Dadurch ist das erfindungsgemäße Verfahren sicherheitstechnisch einfacher in den großtechnischen Maßstab umzusetzen, als Verfahren, bei denen organische Lösungsmittel verwendet werden. Insbesondere sollten der apparative und regelungstechnische Aufwand gegenüber solchen Verfahren, in denen organische Lösungsmittel eingesetzt werden, verringert werden, was zu einer verbesserten Verfahrensökonomie führt. Eine hohe Verfahrensökonomie kann Geänderte Seite zudem durch einfache Aufarbeitungsschritte bzw. eine Rückführung von Edukten erreicht werden. Das erfindungsgemäße Verfahren weist zudem in der Regel ausreichend kurze Verweilzeiten auf, so dass eine kontinuierliche Durchführung möglich ist. Das erfindungsgemäße Verfahren eignet sich insbesondere zur Herstellung von interessanten Nitrilen, die als Zwischenprodukte für die Synthese von Pharmazeutika, Pflanzenschutzmitteln, Lichtschutzmitteln oder Stabilisatoren eingesetzt werden können. So können in einer besonderen Ausführungsform aus Diaminen die entsprechenden Aminonitrile erhalten werden, die wiederum durch Hydrolyse zu den entsprechenden Aminoamiden umgesetzt werden können.

Die Erfindung wird in den nachfolgenden Beispielen erläutert.

### Herstellung des Oxidationskatalysators:

In einem beheizbaren und mit Rührwerk ausgestatteten Fälltopf wurden 1,5 l Wasser vorgelegt und auf 80°C erwärmt. In dieses Fällgefäß wurden im Verlauf einer Stunde eine Metallsalzlösung bestehend aus 877 g Cu(NO₃)₂·5H₂O und 1410 g Al(NO₃)₃·9H₂O in 2000 ml Wasser dosiert. Gleichzeitig mit der Dosierung der Metallsalze erfolgte die Dosierung einer Sodalösung (20 Gew.-% NaOH in Wasser). Die Sodadosierung wurde so gewählt, dass sich im Fällgefäß ein pH-Wert von 6 einstellte. Nach vollständiger Zugabe der Metallsalzlösung wurde weiter Sodalösung zudosiert, bis im Fällgefäß ein pH-Wert von 8 erreicht wurde. Nach Erreichen dieses pH-Wertes wurde die Mischung weitere 15 Minuten gerührt. Der Gesamtverbrauch an Sodalösung betrug 4,4 kg. Die gebildete Suspension wurde filtriert und der Filterrückstand mit Wasser gewaschen, bis das ablaufende Waschwasser weniger als 25 ppm Nitrat enthielt. Das Produkt wurde bei 120°C getrocknet, zu Formkörpern gepresst, calciniert, und anschließend gemahlen und gesiebt. Die Fraktion 0,6-1,6 mm Split wurde im weiteren eingesetzt. Der so erhaltene Katalysator enthielt 61 Gew.-% CuO und 39 Gew.-% Al₂O₃.

### Beispiel 1:

Eine Lösung von 2,2-Dimethylpropandiamin in Wasser (10 Gew.-% 2,2-Dimethylpropandiamin in Wasser) wurde kontinuierlich mit 0,5 ml/min (min: Minute) in einen Verdampfer (200°C) gepumpt und mit 20 Nl/h Luft (Nl: Normliter; h: Stunde) und 20 Nl/h N₂ über einen Rohrreaktor (20 ml) gefahren, der mit 20 ml (13,5g) des Oxidationskatalysators CuO/Aluminiumoxid (Herstellung s.o.) gefüllt war. Der Reaktionsaustrag wurde durch Kondensation aufgefangen.
Nach einer Betriebsdauer von einer Stunde wurde in der kondensierten Lösung folgendes Produktgemisch gefunden: 5,9% Edukt, 48,5% 3-Amino-2,2-dimethylpropionitril (Rest: sonstige Verbindungen).
Die entspricht einer Selektivität von 52% bei einem Umsatz von 94,1%.
Nach einer Betriebsdauer von 3 Stunden ging der Umsatz auf 84% zurück.

Diese Desaktivierung des Oxidationskatalysators konnte durch Ausbau des Oxidationskatalysators und Temperung bei 600°C unter Luft rückgängig gemacht werden. Bei der Wiederverwendung des regenerierten Oxidationskatalysators unter den obenbeschriebenen Reaktionsbedingungen wurde nach 1 Stunde ein Umsatz von 94,4% und eine Selektivität von 46% erzielt.

### Beispiel 2:

Eine wässrige 1-Butylamin-Lösung (10 Gew.-%i 1-Butylamin in Wasser) wurde kontinuierlich mit 0,5 ml/min in einen Verdampfer (200°C) gepumpt und mit 20 Nl/h Luft und 20 Nl/h N2 über einen Rohrreaktor (20 ml) gefahren, der mit 20 ml (14 g) des Oxidationskatalysators CuO/Aluminiumoxid (Herstellung s.o.) gefüllt war.
Der Reaktionsaustrag wurde durch Kondensation aufgefangen. Nach einer Betriebsdauer von vier Stunden wurde in der kondensierten Austragslösung folgendes Produktgemisch gefunden: 13% Edukt, 64,9% 3-Amino-2,2-dimethylpropionitril (Rest: sonstige Verbindungen).
Die entspricht einer Selektivität von 75% bei einem Umsatz von 87%.
Der Versuch wurde nach 6 Stunden beendet. Es wurde keine Deaktivierung des Oxidationskatalysators während der Versuchszeit beobachtet.

### Beispiel 3:

Die Umsetzung von 3-Amino-2,2-dimethyl-propionitril erfolgt analog zu Beispiel 1 der WO-A1-98/31657. Anstelle des Aminonitrils von Cyclopentanons wird jedoch 3-Amino-2,2-dimethyl-propionitril in die Reaktion eingesetzt. Es wird 3-Amino-2,2-dimethylpropionsäureamid erhalten.

## Patentansprüche

1. Verfahren zur Herstellung von Nitrilen, Iminen und/oder Enaminen durch Umsetzung eines primären und/oder sekundären Amins, welches ein Alkylamin, Cycloalkylamin, Dicycloalkylamin, Alkoxyamin, Alkandiamin, Cycloalkyldiamin oder Alkoxydiamin ist, in Gegenwart von Sauerstoff und eines Oxidationskatalysators, **dadurch gekennzeichnet, dass** man ein Amin in die Gasphase überführt und in einem sauerstoffhaltigen Gasstrom bei einer Temperatur von 100 bis 350°C über einen Oxidationskatalysator leitet.

2. Verfahren nach Anspruch 1, **dadurch gekennzeichnet, dass** man das Amin als wässrige Lösung des Amins einsetzt und die Konzentration der wässrigen Lösung des Amins im Bereich von 1 bis 90 Gew.-% liegt.

3. Verfahren nach mindestens einem der Ansprüche 1 bis 2, **dadurch gekennzeichnet, dass** man als sauerstoffhaltigen Gasstrom ein Gemisch von Luft und Stickstoff einsetzt, wobei der Volumenanteil von Luft im Gasstrom im Bereich von 30 bis 70 Vol.-% liegt.

4. Verfahren nach mindestens einem der Ansprüche 1 bis 3, **dadurch gekennzeichnet, dass** das Verfahren kontinuierlich durchgeführt wird.

5. Verfahren nach Anspruch 4, **dadurch gekennzeichnet, dass** die mittlere Verweilzeit im Reaktor im Bereich von 0,01 bis 100 Sekunden liegt.

6. Verfahren nach Anspruch 4 oder 5, **dadurch gekennzeichnet, dass** das Verfahren in einem Festbettreaktor durchgeführt wird und die Katalysatorbelastung in einem Bereich von 0,01 bis 10 kg Amin pro Liter Katalysator und Stunde liegt.

7. Verfahren nach mindestens einem der Ansprüche 1 bis 6, **dadurch gekennzeichnet, dass** die Isolierung der gebildeten Reaktionsprodukte durch Einleitung der Reaktionsprodukte in ein inertes Lösungsmittel (Quench) erfolgt.

8. Verfahren nach mindestens einem der Ansprüche 1 bis 6, **dadurch gekennzeichnet, dass** die Isolierung der gebildeten Reaktionsprodukte durch Auskondensierung der Reaktionsprodukte aus dem Gasstrom erfolgt.

9. Verfahren nach mindestens einem der Ansprüche 1 bis 8, **dadurch gekennzeichnet, dass** nichtreagierte Ausgangsprodukte und/oder der sauerstoffhaltige Gasstrom in den Reaktor zurückgeführt werden.

10. Verfahren nach mindestens einem der Ansprüche 1 bis 9, **dadurch gekennzeichnet, dass** die Oxidationskatalysatoren ein oder mehrere Metalloxide ausgewählt aus der Gruppe CuO, ZnO, V₂O₅, Nb₂O₅, MoO₃ und Re₂O₇ enthalten.

11. Verfahren nach Anspruch 10, **dadurch gekennzeichnet, dass** die Oxidationskatalysatoren ein oder mehrere Trägermaterialien enthalten.

12. Verfahren nach mindestens einem der Ansprüche 1 bis 11, **dadurch gekennzeichnet, dass** der Oxidationskatalysator regeneriert wird.

13. Verfahren zur Herstellung von Aminonitrilen gemäß mindestens einem der Ansprüche 1 bis 12, **dadurch gekennzeichnet, dass** man als Amin ein Alkandiamin, Cycloalkyldiamin oder Alkoxydiamin einsetzt.

14. Verfahren zur Herstellung von 3-Amino-2,2-dimethyl-propionitril gemäß Anspruch 13, **dadurch gekennzeichnet, dass** man als Alkandiamin 2,2-Dimethylpropylendiamin einsetzt.

15. Verfahren zur Herstellung von Aminoamiden, **dadurch gekennzeichnet, dass** man
a) ein Aminonitril durch ein Verfahren gemäß Anspruch 13 herstellt, und
b) das in Stufe a) hergestellte Aminonitril hydrolysiert.

16. Verfahren zur Herstellung von 3-Amino-2,2-dimethyl-propionsäureamid, **dadurch gekennzeichnet, dass** man ein gemäß Anspruch 14 hergestelltes 3-Amino-2,2-dimethyl-propionitril hydrolysiert.

17. Verfahren zur Herstellung von Pharmazeutika, Pflanzenschutzmitteln, Lichtschutzmitteln oder Stabilisatoren, **dadurch gekennzeichnet, dass** man
a) zunächst ein Aminonitril durch ein Verfahren gemäß Anspruch 13 herstellt, oder
b) ein Aminoamid durch ein Verfahren gemäß Anspruch 15 herstellt, und
c) das in Stufe a) erhaltene Aminonitril oder das in Stufe b) erhaltene Aminoamid zu Pharmazeutika, Pflanzenschutzmitteln, Lichtschutzmitteln oder Stabilisatoren umsetzt.

## Claims

1. A process for preparing nitriles, imines and/or enamines by converting a primary and/or secondary amine which is an alkylamine, cycloalkylamine, dicycloalkylamine, alkoxyamine, alkanediamine, cycloalkyldiamine or alkoxydiamine in the presence of oxygen and of an oxidation catalyst, which comprises converting an amine to the gas phase and passing it over an oxidation catalyst in an oxygenous gas stream at a temperature of from 100 to 350°C.

2. The process according to claim 1, wherein the amine is used as an aqueous solution of the amine and the concentration of the aqueous solution of the amine is in the range from 1 to 90% by weight.

3. The process according to at least one of claims 1 and 2, wherein the oxygenous gas stream used is a mixture of air and nitrogen, the proportion by volume of air in the gas stream being in the range from 30 to 70% by volume.

4. The process according to at least one of claims 1 to 3, which is performed continuously.

5. The process according to claim 4, wherein the mean residence time in the reactor is in the range from 0.01 to 100 seconds.

6. The process according to claim 4 or 5, which is performed in a fixed bed reactor and wherein the catalyst hourly space velocity is within a range of from 0.01 to 10 kg of amine per liter of catalyst and hour.

7. The process according to at least one of claims 1 to 6, wherein the reaction products formed are isolated by introducing the reaction products into an inert solvent (quench).

8. The process according to at least one of claims 1 to 6, wherein the reaction products formed are isolated by condensing the reaction products out of the gas stream.

9. The process according to at least one of claims 1 to 8, wherein unconverted starting materials and/or the oxygenous gas stream are recycled into the reactor.

10. The process according to at least one of claims 1 to 9, wherein the oxidation catalysts comprise one or more metal oxides selected from the group of CuO, ZnO, V₂O₅, Nb₂O₅, MoO₃ and Re₂O₇.

11. The process according to claim 10, wherein the oxidation catalysts comprise one or more support materials.

12. The process according to at least one of claims 1 to 11, wherein the oxidation catalyst is regenerated.

13. A process for preparing amino nitriles according to at least one of claims 1 to 12, which comprises using an alkanediamine, cycloalkyldiamine or alkoxy-diamine as the amine.

14. A process for preparing 3-amino-2,2-dimethylpropionitrile according to claim 13, which comprises using 2,2-dimethylpropylenediamine as the alkanediamine.

15. A process for preparing amino amides, which comprises
a) preparing an amino nitrile by a process according to claim 13, and
b) hydrolyzing the amino nitrile prepared in stage a).

16. A process for preparing 3-amino-2,2-dimethylpropionamide, which comprises hydrolyzing 3-amino-2,2-dimethylpropionitrile prepared according to claim 14.

17. A process for producing pharmaceuticals, crop protection compositions, light stabilizers or other stabilizers, which comprises
a) first preparing an amino nitrile by a process according to claim 13,
or
b) preparing an amino amide by a process according to claim 15, and
c) converting the amino nitrile obtained in stage a) or the amino amide obtained in stage b) to pharmaceuticals, crop protection compositions, light stabilizers or other stabilizers.

## Revendications

1. Procédé de fabrication de nitriles, d'imines et/ou d'énamines par mise en réaction d'une amine primaire et/ou secondaire, qui est une alkylamine, une cycloalkylamine, une dicycloalkylamine, une alcoxyamine, une alcanediamine, une cycloalkyldiamine ou une alcoxydiamine, en présence d'oxygène et d'un catalyseur d'oxydation, **caractérisé en ce qu'**une amine est transférée dans la phase gazeuse et conduite dans un courant gazeux contenant de l'oxygène à une température de 100 à 350 °C sur un catalyseur d'oxydation.

2. Procédé selon la revendication 1, **caractérisé en ce que** l'amine est utilisée sous la forme d'une solution aqueuse de l'amine et la concentration de la solution aqueuse de l'amine se situe dans la plage allant de 1 à 90 % en poids.

3. Procédé selon au moins l'une quelconque des revendications 1 à 2, **caractérisé en ce qu'**un mélange d'air et d'azote est utilisé en tant que courant gazeux contenant de l'oxygène, la proportion volumique d'air dans le courant gazeux étant dans la plage allant de 30 à 70 % en volume.

4. Procédé selon au moins l'une quelconque des revendications 1 à 3, **caractérisé en ce que** le procédé est réalisé en continu.

5. Procédé selon la revendication 4, **caractérisé en ce que** le temps de séjour moyen dans le réacteur se situe dans la plage allant de 0,01 à 100 secondes.

6. Procédé selon la revendication 4 ou 5, **caractérisé en ce que** le procédé est réalisé dans un réacteur à lit fixe et le chargement du catalyseur se situe dans une plage allant de 0,01 à 10 kg d'amine par litre de catalyseur et par heure.

7. Procédé selon au moins l'une quelconque des revendications 1 à 6, **caractérisé en ce que** l'isolement des produits de réaction formés a lieu par introduction des produits de réaction dans un solvant inerte (trempe).

8. Procédé selon au moins l'une quelconque des revendications 1 à 6, **caractérisé en ce que** l'isolement des produits de réaction formés a lieu par condensation des produits de réaction à partir du courant gazeux.

9. Procédé selon au moins l'une quelconque des revendications 1 à 8, **caractérisé en ce que** les produits de départ non réagis et/ou le courant gazeux contenant de l'oxygène sont recyclés dans le réacteur.

10. Procédé selon au moins l'une quelconque des revendications 1 à 9, **caractérisé en ce que** les catalyseurs d'oxydation contiennent un ou plusieurs oxydes métalliques choisis dans le groupe constitué par CuO, ZnO, V₂O₅, Nb₂O₅, MoO₃ et Re₂O₇.

11. Procédé selon la revendication 10, **caractérisé en ce que** les catalyseurs d'oxydation contiennent un ou plusieurs matériaux supports.

12. Procédé selon au moins l'une quelconque des revendications 1 à 11, **caractérisé en ce que** le catalyseur d'oxydation est régénéré.

13. Procédé de fabrication d'aminonitriles selon au moins l'une quelconque des revendications 1 à 12, **caractérisé en ce qu'**une alcanediamine, une cycloalkyldiamine ou une alcoxydiamine est utilisée en tant qu'amine.

14. Procédé de fabrication de 3-amino-2,2-diméthylpropionitrile selon la revendication 13, **caractérisé en ce que** la 2,2-diméthylpropylènediamine est utilisée en tant qu'alcanediamine.

15. Procédé de fabrication d'aminoamides, **caractérisé en ce que**
a) un aminonitrile est fabriqué par un procédé selon la revendication 13 et
b) l'aminonitrile fabriqué à l'étape a) est hydrolysé.

16. Procédé de fabrication d'un amide de l'acide 3-amino-2,2-diméthyl-propionique, **caractérisé en ce qu'**un 3-amino-2,2-diméthyl-propionitrile fabriqué selon la revendication 14 est hydrolysé.

17. Procédé de fabrication de produits pharmaceutiques, d'agents phytoprotecteurs, d'agents photoprotecteurs ou de stabilisateurs, **caractérisé en ce que**
a) un aminonitrile est tout d'abord fabriqué par un procédé selon la revendication 13, ou
b) un aminoamide est fabriqué par un procédé selon la revendication 15, et
c) l'aminonitrile obtenu à l'étape a) ou l'aminoamide obtenu à l'étape b) est transformé en produits pharmaceutiques, agents phytoprotecteurs, agents photoprotecteurs ou stabilisateurs.
